# EUROPEAN PATENT APPLICATION

(11) **EP 1 532 978 A1**
(43) Date of publication of application: **25.05.2005**
(21) Application number: 03741175.8
(22) Date of filing: 03.07.2003
(51) Int. Cl.: A61K 31/155, A61K 45/00, A61K 31/335, A61K 31/381, A61K 31/4045, A61P 13/02, A61P 43/00

(54) **REMEDY FOR URINARY FREQUENCY AND URINARY INCONTINENCE**

(30) Priority: 09.07.2002 JP 2002200305
(71) Applicant: FUJISAWA PHARMACEUTICAL CO., LTD., Osaka-shi Osaka 541-8514 (JP)
(72) Inventor: YAMAMOTO, Takao, c/o FUJISAWA PHARMACEUT. CO., LTD, Osaka-shi, Osaka 541-8514 (JP); HANIOKA, Naomi, c/o FUJISAWA PHARMACEUT. CO., LTD, Osaka-shi, Osaka 541-8514 (JP); HAMADA, Kaori, c/o FUJISAWA PHARMACEUT. CO., LTD, Osaka-shi, Osaka 541-8514 (JP)
(74) Representative: Gille Hrabal Struck Neidlein Prop Roos
(86) International application number: PCT/JP2003/008457
(87) International publication number: WO 2004/004701

(57) **Abstract**

A composition for treating frequent urination and/or urinary incontinence comprising a compound having an activity to inhibit Na⁺/H⁺ exchange transporter or its pharmaceutically acceptable salt as an active ingredient.

## Description

### Field of the Invention

The present invention relates to a therapeutic agent for frequent urination and/or urinary incontinence. More specifically, it relates to novel applications of substituted guanidine compounds having inhibiting activity against Na⁺/H⁺ exchange transport. In more detail, the present invention relates to a therapeutic agent for frequent urination and/or urinary incontinence containing the substituted guanidine compounds, applications of the same compounds to therapeutic agents for frequent urination and/or urinary incontinence and a therapeutic method for frequent urination and/or urinary incontinence using the same compounds.

### Background Art

In these days, increasing attention has been paid to frequent urination and/or urinary incontinence and there is a demand for treatments for patients in a wide range of diseases such as neuropathic bladder incident to spinal cord injuries caused by disasters, frequent urination and urinary incontinence caused by after-effects of stroke and nervous degenerative diseases. With the arrival of a graying society, sufferers of these disorders are on the increase more than ever. In normal urination, a function of urine retention is maintained, but if it is disordered, frequent urination and/or urinary incontinence occurs. In this situation, involuntary urine leakage occurs and sanitary problems are caused in everyday life. This has been a serious worry of the patients.

So far, anticholinergic agents or the like have been used as a therapeutic drug for the frequent urination and/or urinary incontinence.

On the other hand, a Na⁺/H⁺ exchange transporter is a protein existing on various kinds of cell membranes and has a function of simultaneously discharging intracellular H⁺ ions out of the cells and taking extracellular Na⁺ ions into the cells. Since it has been known that the transfer of the Na⁺ ions is accompanied by the transfer of water, it is assumed that the Na⁺/H⁺ exchange transporter adjusts intracellular pH and a cell volume.

As applications of compounds having inhibiting activity against the Na⁺/H⁺ exchange transporter, for example, Japanese Patent Kokai No. Hei 9-67340 describes their use for prevention or treatment of brain infarction. Further, Japanese Patent Kokai No. Hei 11-286454 describes their use for treatment of ischemic cerebrovascular diseases.

However, there has not been reported the action of inhibiting the frequent urination and/or urinary incontinence of the compounds having the inhibiting activity against the Na⁺/H⁺ exchange transporter.

Further, examples of the substituted guanidine compounds having the inhibiting activity against the Na⁺/H⁺ exchange transporter are compounds described in, for example, Japanese Patent Kokai No. Hei 8-208602, European Patent No. 787728, Japanese Patent Kokai No. Hei 9-291076, Japanese Patent Kokai No. Hei 6-228082, Japanese Patent Kokai No. Hei 10-503770 and WO99/55690.

### Disclosure of the Invention

As a result of intensive researches for finding an agent which inhibits the frequent urination and/or urinary incontinence, the inventors of the present invention have found that the substituted guanidine compounds having the inhibiting activity against the Na⁺/H⁺ exchange transporter have an excellent action of inhibiting the frequent urination and/or urinary incontinence, and whereby the present invention has been achieved.

More specifically, the inventors of the present invention have found on a frequent urination model, a rhythmic bladder contraction model of a rat, that the substituted guanidine compounds having the inhibiting activity against the Na⁺/H⁺ exchange transporter have an excellent action of decreasing the bladder contractile frequency without significantly affecting the bladder contractile force, and whereby the present invention has been achieved.

### Best Mode for Carrying Out the Invention

Hereinafter, the present invention will be explained in detail.

The substituted guanidine compounds having the inhibiting activity against the Na⁺/H⁺ exchange transporter and pharmaceutically acceptable salts thereof used in the present invention are not particularly limited as long as they have the inhibiting activity against the Na⁺/H⁺ exchange transporter. For example, those described in the following (a) to (f) may be used.

### (A) Substituted guanidine compounds represented by the general formula (Ia) :

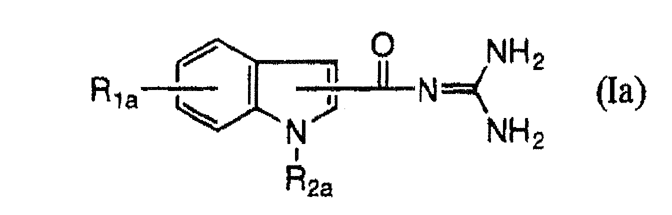

wherein R₁ₐ represents a hydrogen atom, a halogen atom, a nitro group, a carboxy group, an alkyl group, a substituted alkyl group, an alkenyl group, an alkynyl group, a cycloalkyl group, an acyl group, an alkoxycarbonyl group, an aromatic group, a group represented by the formula -OR₃ₐ, -NR₄ₐR₅ₐ, -SO₂NR₄ₐR₅ₐ or -S(O)ₙR₆ₐ,
in which R₃ₐ represents a hydrogen atom, an alkyl group, a substituted alkyl group, a cycloalkyl group, an aromatic group, or a group represented by the formula -CH₂R₇ₐ, where R₇ₐ represents a alkenyl group or a alkynyl group,
R₄ₐ and R₅ₐ represent independently a hydrogen atom, an alkyl group, a substituted alkyl group, a cycloalkyl group, an acyl group, an aromatic group or a group represented by the formula -CH₂R₈ₐ, where
R₈ₐ represents alkenyl group or an alkynyl group, or
R₄ₐ and R₅ₐ are combined together to form a 5 to 7 membered saturated cyclic amino group in which another hetero atom may be optionally contained,
R₆ₐ represents a alkyl group, a substituted alkyl group or an aryl group, and
n represents 0, 1 or 2,
or a group of the formula: 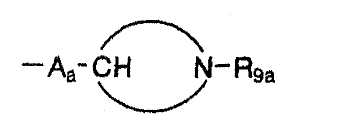 wherein the ring represents a 3 to 8 membered saturated heterocyclic ring containing a nitrogen atom and Aₐ represents an oxygen atom or a group of the formula -S(O)ₙ- or -N(R₁₀ₐ)-, in which R₁₀ₐ is hydrogen atom or alkyl group, and n is 0, 1 or 2;
R₉ₐ represents a hydrogen atom, an alkyl group or a substituted alkyl group, which may be further substituted with suitable substituent(s);
R₂ₐ represents a hydrogen atom, a hydroxy group, an alkyl group, a substituted alkyl group, a cycloalkyl group, an alkoxy group, an aromatic group, or a group represented by the formula -CH₂R₇ₐ, where R₇ₐ represents an alkenyl group or an alkynyl group;
the group R₁ₐ and guanidinocarbonyl group being optionally combined to either one of the 6-membered ring moiety and 5-membered ring moiety of the indole ring;
or pharmaceutically acceptable salts thereof.

With regard to these compounds, the halogen atom may be, for example, fluorine, chlorine and bromine atoms.

The alkyl group may be linear or branched C₁-C₈ alkyl groups such as methyl, ethyl, propyl, isopropyl, butyl, 2-butyl, 2-methylpropyl, 1,1-dimethylethyl, pentyl, hexyl, heptyl or octyl.

The alkenyl group may be C₂-C₆ alkenyl groups such as vinyl, allyl, propenyl, butenyl, pentenyl or hexenyl.

The alkynyl group may be C₂-C₆ alkynyl groups such as ethynyl, propargyl, butynyl or pentynyl.

The cycloalkyl group may be C₃-C₇ cycloalkyl groups such as cyclopropyl, cyclobutyl, cyclopentyl, cyclohexyl or cycloheptyl.

The acyl group may be linear or branched C₁-C₈ alkanoyl groups, such as formyl, acetyl, propanoyl or 2-methylpropanoyl, a C₈-C₁₂ arylalkanoyl groups such as phenylacetyl or phenylpropanoyl, or C₇-C₁₁ aroyl groups such as benzoyl, 1-naphthoyl or 2-naphthoyl.

The alkoxycarbonyl group may be linear or branched C₂-C₇ alkoxycarbonyl groups such as methoxycarbonyl, ethoxycarbonyl, propoxycarbonyl or 2-propoxycarbonyl.

The aromatic group includes optionally substituted aryl group and optionally substituted hetero-aryl group. The aryl group may be C₆-C₁₀ aryl groups such as phenyl or naphthyl, and the hetero-aryl group may be 5 or 6 membered hetero-aryl groups containing 1 to 4 nitrogen atoms, 5 or 6 membered hetero-aryl groups containing 1 or 2 nitrogen atoms and one of oxygen or sulfur atom, such as 2-, 3- or 4-pyridyl, imidazolyl, triazolyl, tetrazolyl, 2- or 3-furyl, 2- or 3-thienyl, 1-, 3- or 4-oxazolyl, or 3-, 4- or 5-isoxazolyl.

The substituent in the substituted aryl group and the substituted hetero-aryl group includes a halogen atom, a nitro group, a carboxy group, an alkyl group, a substituted alkyl group, an alkoxycarbonyl group, and a group represented by the formula -OR₃ₐ, -NR₄ₐR₅ₐ, -CONR₄ₐ, R₅ₐ, -SO₂NR₄ₐR₅ₐ or -S(O)ₘR₆ₐ.

In case where R₁ is a group represented by the formula -OR₃ₐ and R₃ₐ is an aryl group, examples of the representative group for -OR₃ₐ is a phenoxy group and a substituted phenoxy group. Examples of the substituted phenoxy group may be a phenoxy group substituted, for example, with a nitro group, -NR₄ₐR₅ₐ group, wherein R₄ₐ and R₅ₐ are each for example a hydrogen atom or an alkyl group, or with a substituted alkyl group, wherein the substituent is for example a hydroxy group or -NR₄ₐR₅ₐ group. Particularly the substituted phenoxy group may be, for example, o-, m- or p-nitrophenoxy, o-, m- or p-aminophenoxy, o-, m- or p-(dimethylamino)phenoxy, o-, m- or p-(aminomethyl)phenoxy, or o-, m- or p-(dimethylaminomethyl)phenoxy.

The alkoxy group may be C₁-C₆ linear or branched alkoxy groups such as methoxy, ethoxy, isopropoxy or tert-butoxy.

The saturated 5 to 7 membered cyclic amino group, formed by combining R₄ₐ and R₅ₐ which optionally contains another hetero atom in the ring, may be for example 5 to 7 membered cyclic group containing 1 to 3 nitrogen atoms or 5 to 7 membered cyclic group containing one of nitrogen atom and one of oxygen atom, particularly 1-pyrrolidinyl, piperidino, 1-piperazinyl, morpholino, 4-methylpiperazinyl.

The substituent in the substituted alkyl group includes a halogen atom, a hydroxyl group, a cyano group, a carboxy group, an alkoxy group, an alkoxycarbonyl group, an acyl group, an aromatic group, or a group represented by the formula -CONR₄ₐR₅ₐ, wherein R₄ₐ and R₅ₐ represent independently hydrogen atom or alkyl group, or R₄ₐ and R₅ₐ are combined together to form a 5 to 7 membered saturated cyclic amino group optionally containing another hetero atom in the ring, the formula -NR₄ₐR₅ₐ or the formula: 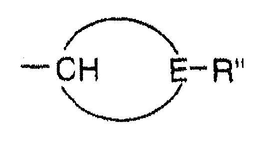 wherein E represents a nitrogen atom or CH group ; R" represents a hydrogen atom, an alkyl group or a substituted alkyl group; and the ring represents 3 to 8 membered saturated aliphatic ring or saturated heterocyclic ring containing a nitrogen atom.

The substituent in the substituted alkyl group may be a halogen atom, a hydroxy group, a carboxy group, a cycloalkyl group, an alkoxy group, an alkoxycarbonyl group, an acyl group, an aromatic group, or a group represented by the formula -CONR₄ₐR₅ₐ or -NR₄ₐR₅ₐ.

The substituent in the substituted alkyl group for R₄ₐ and R₅ₐ may be a hydroxy group, a carboxy group, a cycloalkyl group, an alkoxy group, an alkoxycarbonyl group, an acyl group, an aryl group or a group represented by the formula -CONR₄ₐR₅ₐ or -NR₄ₐR₅ₐ. The alkyl moiety in the substituted alkyl group may be the same as the alkyl group mentioned in the above.

Said substituted alkyl group may be, for example, C₁-C₅ alkyl groups substituted with a C₃-C₆ cycloalkyl, a C₁-C₅ polyhaloalkyl group, a C₁-C₆ hydroxyalkyl group, a C₂-C₆ alkoxyalkyl group, a C₂-C₆ cyanoalkyl group, a C₂-C₆ carboxyalkyl group, a C₃-C₈ alkoxycarbonylalkyl group, a C₃-C₈ alkanoylalkyl group, a C₈-C₁₆ aroylalkyl group, optionally substituted phenyl- or naphthyl-C₁-C₅ alkyl group, carbamoyl-C₁-C₃ alkyl group in which the nitrogen atom may be optionally substituted with one or two C₁-C₃ alkyl, amino-C₁-C₅ alkyl groups in which the nitrogen atom may be optionally substituted with one or two C₁-C₃ alkyl or C₇-C₁₁ aralkyl, or 5 to 7 membered saturated cyclic amino-C₁-C₃ alkyl groups.

Particularly the substituted alkyl group for R₁ₐ may be C₁-C₃ polyhaloalkyl groups such as trifluoromethyl, trifluoroethyl or trichloromethyl, or C₁-C₆ hydroxyalkyl groups such as hydroxymethyl, hydroxyethyl or 1-hydroxyethyl, or C₁-C₅ aminoalkyl groups such as aminomethyl, aminoethyl or 1-aminoethyl. The substituted alkyl group for R₂ₐ may be C₁-C₆ hydroxyalkyl groups such as hydroxyethyl, hydroxypropyl, hydroxybutyl, 2-hydroxypropyl or 3,4-dihydroxybutyl, or C₁-C₆ alkoxyalkyl groups such as methoxyethyl, ethoxyethyl or methoxypropyl, or C₂-C₆ carboxyalkyl groups such as carboxyethyl or carboxypropyl, or C₃-C₇ alkoxycarbonylalkyl groups such as methoxycarbonylmethyl, ethoxycarbonylmethyl or methoxycarbonylethyl, or phenyl- or naphthyl-C₁-C₅ alkyl groups such as benzyl, phenylethyl, phenylpropyl, phenylbutyl, or 1- or 2-naphthylmethyl, wherein the phenyl or naphthyl moiety may have substituent such as a halogen atom, a nitro group, an amino group, a hydroxy group, a C₁-C₃ alkyl group or a C₁-C₃ alkoxy group, or a carbamoyl-C₁-C₃ alkyl group in which the nitrogen atom may be optionally substituted with one or two C₁-C₃ alkyl such as carbamoylmethyl, carbamoylethyl or dimethylcarbamoylmethyl, or an amino-C₁-C₅ alkyl group in which the nitrogen atom may be optionally substituted with one or two C₁-C₃ alkyl such as aminoethyl, aminopropyl, dimethylaminoethyl, dimethylaminopropyl or diethylaminoethyl. The substituted alkyl group for R₃ₐ and R₆ₐ may be C₁-C₆ hydroxyalkyl groups such as hydroxyethyl, hydroxypropyl, 2-hydroxypropyl, hydroxybutyl or 2,3-dihydroxypropyl, or C₂-C₆ carboxyalkyl groups such as carboxymethyl or carboxyethyl, or phenyl-C₁-C₅ alkyl groups such as benzyl, phenylethyl or phenylpropyl, or carbamoyl-C₁-C₃ alkyl groups such as carbamoylmethyl or carbamoylethyl, or amino-C₁-C₅ alkyl groups in which the nitrogen atom may be optionally substituted with one or two C₁-C₃ alkyl or C₇-C₁₁ aralkyl such as aminoethyl, aminopropyl, dimethylaminoethyl, dimethylaminopropyl or benzylmethylaminoethyl, or 5 to 7 membered saturated cyclic amino-C₁-C₃ alkyl groups such as 1-pyrrolidinylethyl or piperidinoethyl.

The substituted alkyl group for R₄ₐ and R₅ₐ may be phenyl-C₁-C₅ alkyl groups such as phenylethyl.

The group represented by the formula -S(O)ₙR₆ₐ may be, for example, C₁-C₈ alkanesulfonyl groups such as methanesulfonyl group, ethanesulfonyl group, propanesulfonyl group, isopropanesulfonyl group, or the corresponding alkylsulfinyl group or alkylthio group.

The group represented by the formula: 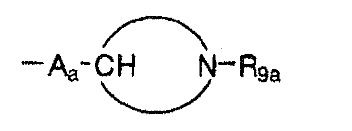 may be, for example, a group represented by the formula: 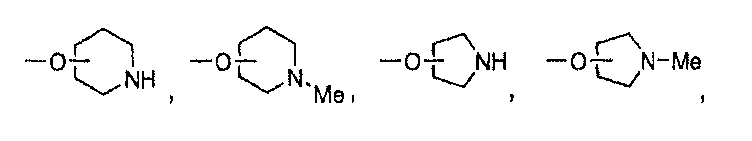 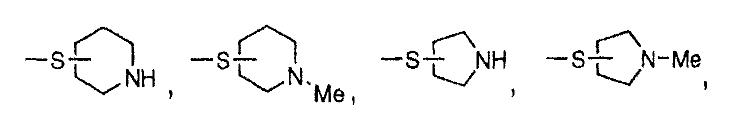 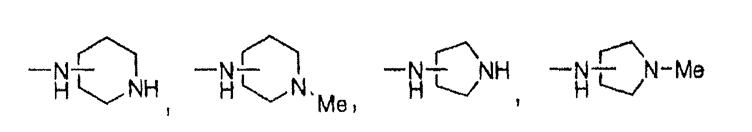 and preferably may be (piperidin-3-yl)oxy, (piperidin-4-yl)oxy, (1-methylpiperidin-3-yl)oxy, (1-methylpiperidin-4-yl)oxy, (pyrrolidin-3-yl)oxy, (1-methylpyrrolidin-3-yl)oxy, (piperidin-3-yl)thio, (piperidin-4-yl)thio, (1-methylpiperidin-3-yl)thio, (1-methylpiperidin-4-yl)thio, (pyrrolidin-3-yl)thio, (1-methylpyrrolidin-2-yl)thio, (piperidin-3-yl)amino, (piperidin-4-yl)amino, (1-methylpiperidin-3-yl)amino, (1-methylpiperidin-4-yl)amino, (pyrrolidin-3-yl)amino or (1-methylpyrrolidin-3-yl)amino.

### (b) Substituted guanidine compound represented by the general formula (Ib):

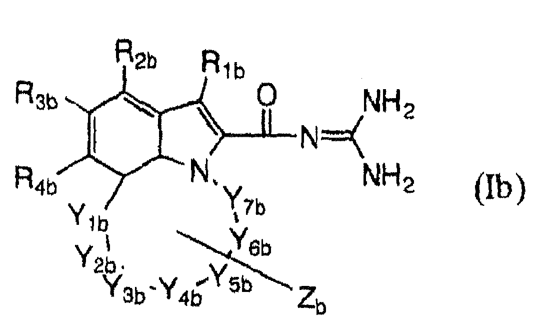

wherein R_{1b}, R_{2b}, R_{3b} and R_{4b} represent independently a hydrogen atom, a halogen atom, a nitro group, a carboxy group, an alkyl group, a substituted alkyl group, a cycloalkyl group, a cycloalkenyl group, a saturated heterocyclic group, an alkoxycarbonyl group, an aromatic group, an acyl group, or a group represented by the formula -OR_{5b}, -N(R_{6b})R_{7b}, -CON(R_{6b})R_{7b}, -SO₂N(R_{6b})R_{7b}, -S(O)ₙR_{8b}, -Q_{b}-R_{9b} or the formula: 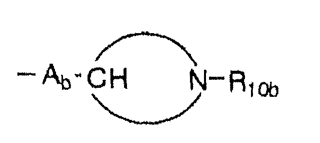 wherein R_{5b} represents a hydrogen atom, an alkyl group, a substituted alkyl group, a cycloalkyl group, a cycloalkenyl group, a saturated heterocyclic group or an aromatic group;
R_{6b} and R_{7b} represent independently a hydrogen atom, an alkyl group, a substituted alkyl group, a cycloalkyl group, a cycloalkenyl group, a saturated heterocyclic group, an aromatic group, an acyl group or a group -Q_{b}-R_{9b}, or
R_{6b} and R_{7b} are combined together to form with the nitrogen atom to which they are combined a 5 to 7 membered saturated cyclic amino group which may contain another hetero atom in the ring, in which the said cyclic amino group may be substituted with one or more hydroxy groups, alkyl groups, substituted alkyl groups, or a group -OR_{5b};
R_{8b} represents an alkyl group, a substituted alkyl group or an aromatic group;
Q_{b} represents an optionally substituted lower alkylene group;
R_{9b} represents an optionally substituted vinyl group or optionally substituted ethynyl group;
A_{b} represents an oxygen atom, -S(O)ₙ- or -N(R_{11b})-, wherein R_{11b} represents a hydrogen atom, an alkyl group, a substituted alkyl group, a cycloalkyl group, a saturated heterocyclic group, an aromatic group, an acyl group or -Q_{b}-R_{9b};
R_{10b} represents a hydrogen atom, an alkyl group, a substituted alkyl group, an acyl group or -Q_{b}-R_{9b};
the ring represents 3 to 8 membered saturated heterocyclic group consisting of a nitrogen atom and carbon atoms;
Y_{1b}, Y_{2b}, Y_{3b}, Y_{4b}, Y_{5b}, Y_{6b}, Y_{7b} are the same or different and represent a single bond, -CH₂-, -O-, -CO-, -C(=C(R_{12b})R_{13b})-, -S(O)ₙ-, or -N(R_{11b})-,
wherein R_{12b} and R_{13b} represent independently a hydrogen atom, an alkyl group, a substituted alkyl group, an alkenyl group, an alkynyl group, a cycloalkyl group, a cycloalkenyl group, a saturated heterocyclic group, a halogen atom, an carboxy group, an alkoxycarbonyl group, an aromatic group, an acyl group or a group -OR_{5b}, -N(R_{6b})R_{7b}, -CON(R_{6b})R_{7b}, -S(O)ₙR_{8b} or -Q_{b}-R_{9b};
n is an integer of 0, 1 or 2,
or adjacent two groups represent together -CH=CH-, provided that among Y_{1b} to Y_{7b}, at least two of them represent groups other than single bond;
Z_{b}, which may be absent or may be one or more and substitutes for hydrogen atom attaching to the carbon atom constituting the ring Y_{1b} to Y_{7b}, are the same or different, each represents an alkyl group, a substituted alkyl group, an alkenyl group, an alkynyl group, a cycloalkyl group, a cycloalkenyl group, a saturated heterocyclic group, a halogen atom, an carboxy group, an alkoxycarbonyl group, an aromatic group, an acyl group or a group -OR_{5b}, -N(R_{6b})R_{7b}, -S(O)ₙR_{8b}, -C(O)N(R_{6b})R_{7b}, or -Q_{b}-R_{9b}, provided that Z_{b} is not -N(R_{6b})R_{7b} or -S(O)ₙR_{8b} when Z_{b} substitutes for the hydrogen atom of -CH=CH-;
or its pharmaceutically acceptable salt.

In the compound (Ib), the alkyl group may be linear or branched C₁-C₈ alkyl groups such as methyl, ethyl, propyl, isopropyl, butyl, 2-butyl, 2-methylpropyl, 1,1-dimethylethyl, pentyl, hexyl, heptyl or octyl.

The cycloalkyl group may be 3 to 8 membered cycloalkyl group, which may be unsubstituted or substituted with 1 to 4 alkyl group, substituted alkyl group, hydroxy group or a group -OR_{5b} such as cyclopropyl, cyclobutyl, cyclopentyl, cyclohexyl, cycloheptyl, 2-methylcyclopentyl, 3-methylcyclopentyl, 2-methylcyclohexyl, 3-methylcyclohexyl, 4-methylcyclohexyl, 2-hydroxycyclopentyl, 3-hydroxycyclopentyl, 2-hydroxycyclohexyl, 3-hydroxycyclohexyl, 4-hydroxycyclohexyl, 2-(hydroxymethyl)cyclopentyl, 3-(hydroxymethyl)cyclopentyl, 2-(hydroxymethyl)cyclohexyl, 3-(hydroxymethyl)cyclohexyl, 4-(hydroxymethyl)cyclohexyl, 2-(aminomethyl)cyclopentyl, 3-(aminomethyl)cyclopentyl, 2-(aminomethyl)cyclohexyl, 3-(aminomethyl)cyclohexyl, 4-(aminomethyl)cyclohexyl, 2-(methoxymethyl)cyclopentyl, 3-(methoxymethyl)cyclopentyl, 2-(methoxymethyl)cyclohexyl, 3-(methoxymethyl)cyclohexyl or 4-(methoxymethyl)cyclohexyl.

The cycloalkenyl group may be 3 to 8 membered cycloalkenyl group containing a double bond, which is unsubstituted or substituted with 1 to 4 alkyl groups, substituted alkyl groups, hydroxy groups or a group -OR_{5b}, for example, 1-cyclopentenyl, 2-cyclopentenyl, 3-cyclopentenyl, 1-cyclohexenyl, 2-cyclohexenyl or 3-cyclohexenyl.

The saturated heterocyclic group may be 3 to 8 membered saturated heterocyclic group containing one of oxygen atom or one of sulfur atom, which is unsubstituted or substituted with 1 to 4 alkyl groups, substituted alkyl groups, hydroxy groups or a group -OR_{5b}, for example, 2-tetrahydrofuranyl, 3-tetrahydrofuranyl, 2-tetrahydro-2H-pyranyl or 4-tetrahydro-4H-pyranyl.

The halogen atom may be, for example, fluorine, chlorine and bromine atoms.

The alkoxycarbonyl group may be linear or branched C₂-C₆ alkoxycarbonyl groups such as methoxycarbonyl, ethoxycarbonyl, propoxycarbonyl or 2-propoxycarbonyl.

The aryl group includes an optionally substituted aryl group and an optionally substituted heteroaryl group.

The aryl group may be C₆-C₁₀ aryl groups, for example, phenyl or naphthyl, and the hetero-aryl group may be 5 or 6 membered hetero-aryl groups containing 1 to 4 nitrogen atoms, or containing 0-2 nitrogen atoms and one of oxygen atom or one of sulfur atom, such as 2-, 3-, 4-pyridyl, pyrrolyl, isoimidazolyl, triazolyl, tetrazolyl, 2-, 3-furyl, 2-, 3-thienyl, 1-, 3-, 4-oxazolyl, or 3-, 4- or 5-isoxazolyl.

The substituent in the substituted aryl group and the substituted hetero-aryl group may be a halogen atom, a nitro group, a carboxy group, an alkyl group, a substituted alkyl group, an alkoxycarbonyl group, or a group represented by the formula -OR_{5b}, -N(R_{6b})R_{7b}, -CON(R_{6b})R_{7b}, -SO₂N(R_{6b})R_{7b} or -S(O)nR_{8b}.

In the case where R_{1b}, R_{2b}, R_{3b} or R_{4b} is a group represented by the formula -OR_{5b} and R_{5b} is an aryl group, representative examples of the group -OR_{5b} are a phenoxy group and a substituted phenoxy group. Examples of the substituted phenoxy group may be a phenoxy group substituted with, for example, a nitro group, -N(R_{6b})R_{7b} group, (where R_{6b} and R_{7b} are, for example, a hydrogen atom or an alkyl group), an alkyl group, a substituted alkyl group, (where the substituent is, for example, hydroxy group, -N(R_{6b})R_{7b} group.) or the like.

Further particular substituted phenoxy group may be, for example, o-, m- or p-nitrophenoxy, o-, m- or p-aminophenoxy, o-, m- or p-(dimethylamino)phenoxy, o-, m- or p-(aminomethyl)phenoxy, or o-, m- or p-(dimethylaminomethyl)phenoxy.

The alkoxy group may be linear or branched C₁-C₆ alkoxy groups such as methoxy, ethoxy, isopropoxy or tert-butoxy.

The 5 to 7 membered saturated cyclic amino group which is formed by R_{6b} and R_{7b} together with the nitrogen atom to which they are combined and which optionally contains another hetero atom in the ring may be, for example, 5 to 7 membered cyclic group containing 1 to 3 nitrogen atoms or 5 to 7 membered cyclic group containing one of nitrogen atom and one of oxygen atom, more particularly 1-pyrrolidinyl, 1-piperidino, 1-piperazinyl, morpholino or 1-(4-methyl)piperazinyl.

The substituent in the substituted alkyl group includes a halogen atom, a hydroxy group, a carboxy group, an alkoxy group, a cycloalkyl group, a cyano group, an alkoxycarbonyl group, an acyl group, an aryl group or a group of the formula -CONR_{pb}R_{qb}, in which R_{pb} and R_{qb} represent independently a hydrogen atom or an alkyl group, or R_{pb} and R_{qb} are combined together to form a 5 to 7 membered saturated cyclic amino group optionally containing another hetero atom in the ring, a group of the formula -N(R_{6b})R_{7b} or the formula: 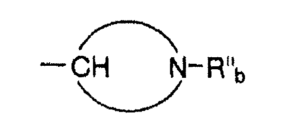 wherein R"_{b} represents a hydrogen atom, an alkyl group or a substituted alkyl group, and the ring represents a 3 to 8 membered saturated hetero-ring containing a nitrogen atom.

Particularly the substituent in the case where R_{1b}, R_{2b}, R_{3b}, R_{4b}, R_{5b}, R_{8b}, R_{11b}, R_{12b} or Z_{b} is a substituted alkyl group may be a halogen atom, a hydroxy group, a carboxy group, a cycloalkyl group, an alkoxy group, an alkoxycarbonyl group, an acyl group, an aromatic group or a group represented by the formula -CONR_{pb}R_{qb} or -N(R_{6b})R_{7b}, and the substituent in the case where R_{6b}, R_{7b}, R_{9b}, or R_{10b} is a substituted alkyl group may be a hydroxy group, a carboxy group, a cycloalkyl group, an alkoxy group, an alkoxycarbonyl group, an acyl group, an aryl group, or a group represented by the formula -CONR_{pb}R_{qb} or -NR_{pb}R_{qb}. Further the alkyl moiety of the substituted alkyl group can be the same one as the above-mentioned alkyl group.

Such a substituted alkyl group may be for example a C₁-C₅ alkyl group substituted with C₃-C₆ cycloalkyl, a C₁-C₅ poly-haloalkyl group, a C₁-C₆ hydroxyalkyl group, a C₂-C₆ alkoxyalkyl group, a C₂-C₆ cyanoalkyl group, a C₂-C₆ carboxyalkyl group, a C₃-C₈ alkoxycarbonylalkyl group, a C₃-C₈ alkanoylalkyl group, a C₈-C₁₆ aroylalkyl group, optionally substituted phenyl- or naphthyl-C₁-C₅ alkyl group, a carbamoyl-C₁-C₃ alkyl group optionally substituted with C₁-C₃ alkyl, an amino-C₁-C₅ alkyl group optionally substituted with C₁-C₃ alkyl or C₇-C₁₁ aralkyl, or a 5 to 7 membered saturated cyclic amino-C₁-C₃ alkyl group, or the like.

Representative substituted alkyl group may be C₁-C₃ poly-halo alkyl groups such as trifluoromethyl, trifluoroethyl or trichloromethyl, C₁-C₆ hydroxyalkyl groups such as hydroxymethyl, hydroxyethyl or 1-hydroxyethyl, C₁-C₅ aminoalkyl groups such as aminomethyl, aminoethyl or 1-aminoethyl, C₁-C₆ alkoxyalkyl groups such as methoxyethyl, ethoxyethyl or methoxypropyl, C₂-C₆ carboxyalkyl groups such as carboxyethyl or carboxypropyl, C₃-C₇ alkoxycarbonylalkyl groups such as methoxycarbonylmethyl, ethoxycarbonylmethyl or methoxycarbonylethyl, phenyl- or naphthyl-C₁-C₅ alkyl groups such as benzyl, phenylethyl, phenylpropyl, phenylbutyl, or 1- or 2-naphthylmethyl, wherein the phenyl moiety and the naphthyl moiety may have substituent such as a halogen atom, a nitro group, an amino group, a hydroxy group, a C₁-C₃ alkyl group or C₁-C₃ alkoxy group, or carbamoyl-C₁-C₃ alkyl groups optionally substituted with C₁-C₃ alkyl groups such as carbamoylmethyl, carbamoylethyl or dimethylcarbamoylmethyl, or amino-C₁-C₅ alkyl groups optionally substituted with C₁-C₃ alkyl or C₇-C₁₁ aralkyl such as aminoethyl, aminopropyl, dimethylaminoethyl, dimethylaminopropyl, diethylaminoethyl or N-methyl-N-benzylaminoethyl, or 5 to 7 membered saturated cyclic amino-C₁-C₃ alkyl groups such as 1-pyrrolidinylethyl or piperidinoethyl, or the like. In the groups R_{6b} and R_{7b}, phenyl-C₁-C₅ alkyl group such as phenylethyl, can be mentioned.

The substituent in the lower alkylene group for Q_{b} and the vinyl group and ethynyl group for R_{9b} may be, for example, a carboxy group, an alkyl group, a substituted alkyl group, a cycloalkyl group, a cycloalkenyl group, a saturated heterocyclic group, an alkoxycarbonyl group, an aryl group or a group represented by the formula -CON(R_{6b})R_{7b} or the like.

The lower alkylene group may be C₁-C₆ alkylene groups such as methylene, ethylene, trimethylene, tetramethylene, pentamethylene or hexamethylene.

The acyl group may be C₁-C₆ alkanoyl groups such as formyl, acetyl or propanoyl, or C₄-C₇ cycloalkanecarbonyl groups such as cyclopropanecarbonyl, cyclobutanecarbonyl, cyclopentanecarbonyl or cyclohexanecarbonyl, or C₄-C₇ cycloalkenecarbonyl groups such as cyclopentenecarbonyl or cyclohexenecarbonyl, or C₇-C₁₁ aroyl groups such as benzoyl, toluoyl or naphthoyl, or saturated heterocyclic-carbonyl groups having 5- or 6-membered heterocyclic ring containing 1-2 hetero atom(s) selected from nitrogen atom, oxygen atom and sulfur atom such as 2-piperidinecarbonyl or 3-morpholinecarbonyl, or acyl group having 5-or 6-membered aromatic heterocyclic ring containing 1-2 hetero atom(s) selected from nitrogen atom, oxygen atom and sulfur atom such as furoyl, thenoyl, nicotinoyl, isonicotinoyl, or the like.

The 5 to 7 membered saturated cyclic amino group formed by combination of R_{pb} and R_{qb} and optionally containing another hetero atom in the ring may be the same as the above cyclic amino group formed by combination of R_{6b} and R_{7b}.

The group represented by the formula -S(O)ₙR_{8b} may be C₁-C₈ alkanesulfonyl groups such as methanesulfonyl, ethanesulfonyl, propanesulfonyl, isopropanesulfonyl, and the corresponding alkanesulfinyl group and alkylthio group.

The group represented by the formula: 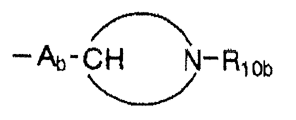 may be, for example, a group represent by the formula: 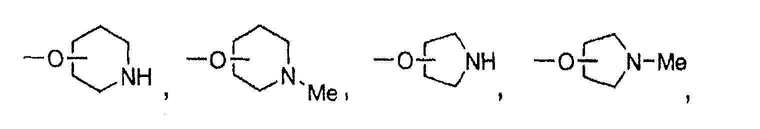 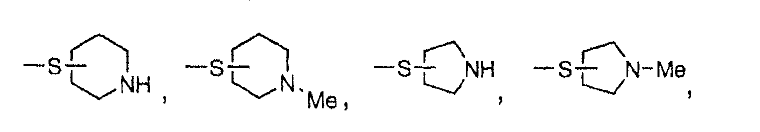 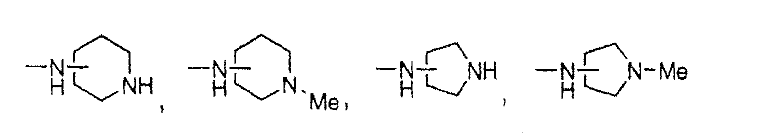 and preferably may be (piperidin-3-yl)oxy, (piperidin-4-yl)oxy, (1-methylpiperidin-3-yl)oxy, (1-methylpiperidin-4-yl)oxy, (pyrrolidin-3-yl)oxy, (1-methylpyrrolidin-3-yl)oxy, (piperidin-3-yl)thio, (piperidin-4-yl)thio, (1-methylpiperidin-3-yl)thio, (1-mthylpiperidin-4-yl)thio, (pyrrolidin-3-yl)thio, (1-methylpyrrolidin-2-yl)thio, (piperidin-3-yl)amino, (piperidin-4-yl)amino, (1-methylpiperidin-3-yl)amino, (1-methylpiperidin-4-yl)amino, (pyrrolidin-3-yl)amino or (1-methylpyrrolidin-3-yl)amino.

The alkenyl group may be C₂-C₆ alkenyl groups such as vinyl, allyl, propenyl, 2-propenyl, butenyl, pentenyl or hexenyl.

The alkynyl group may be C₂-C₆ alkynyl groups such as ethynyl, propargyl, butynyl or pentynyl.

The groups Y_{1b}, Y_{2b}, Y_{3b}, Y_{4b}, Y_{5b}, Y_{6b} and Y_{7b} may be, for example, one of the followings.
1. Among Y_{1b} to Y_{7b}, any one of them represents -CH₂-, -O-, -CO-, -C(=C(R_{12b})R_{13b})-, -S(O)ₙ- or -N(R_{11b})-, another one represents -CH₂-, and the other five are the same or different and represent each a single bond or -CH₂-.
   Further particularly, the followings can be mentioned.
   1-1. Y_{1b} represents -CH₂-, -O-, -CO-, -C(=C(R_{12b})R_{13b})-, -S(O)ₙ- or -N(R_{11b})-, Y_{2b} represents -CH₂-, and Y_{3b}-Y_{7b} are the same or different and each represents a single bond or -CH₂-.
   1-2. Y_{7b} represents -O-, -CO- or -C(=C(R_{12b})R_{13b})-, Y_{6b} represents -CH₂- and Y_{1b} to Y_{5b} are the same or different and each represents a single bond or -CH₂-.
   1-3. Y_{1b} and Y_{7b} represent -CH₂-, any one of Y_{2b}, Y_{3b}, Y_{4b}, Y_{5b} and Y_{6b} represents -CH₂-, -O-, -C(=C(R_{12b})R_{13b})-, -S(O)ₙ- or-N(R_{11b})-, and the other four are the same or different and each represents a single bond or -CH₂-. 1-4. Y_{1b} represents -CH₂-, -O-, -CO-, -C(=C(R_{12b})R_{13b})-, -S(O)ₙ- or -N(R_{11b})-, each of Y_{2b} to Y_{4b} represents -CH₂-, and Y_{5b} and Y_{6b} represent each a single bond.
2. Any adjacent two of Y_{1b} to Y₆b are combined together to form -CH=CH-, and the other four are the same or different and each of them represents a single bond or -CH₂-, and Y_{7b} represents a single bond, -O-, -CO-, -C(=C(R_{12b})R_{13b})- or -CH₂-.
   More particularly, the followings can be mentioned.
   2-1. -Y_{1b}-Y_{2b}- represents -CH=CH-.
   2-2. Y_{1b} represents -CH₂- and -Y_{2b}-Y_{3b}- represents -CH=CH-.
   2-3. Y_{1b} and Y_{2b} represent each -CH₂-, and -Y_{3b}-Y_{4b}- represents -CH=CH-.
   2-4. Y_{1b}, Y_{2b} and Y_{3b} represent each -CH₂-, and -Y_{4b}-Y_{5b}- represents -CH=CH-.
3. Y_{1b} represents -O- or -N(R_{11b})-, any one of Y_{2b} to Y_{7b} represents -CO-, and the other five are the same or different and each of them represents a single bond or -CH₂-.
   More particularly, the followings can be mentioned.
   3-1. Y_{2b} represents -CO-.
   3-2. Y_{2b} represents -CH₂-, and Y_{3b} represents -CO-.
   3-3. Y_{2b} and Y_{3b} represent -CH₂-, and Y_{4b} represents -CO-.
   3-4. Y_{2b}, Y_{3b} and Y_{4b} represent each -CH₂-, and Y_{5b} represents -CO-.
   3-5. Y_{2b}, Y_{3b}, Y_{4b} and Y_{5b} represent each -CH₂-, and Y_{6b} represents -CO-.

Preferably two to five among Y_{1b} to Y_{7b}, especially two to four of them represent each single bond, and the remains represent the other groups than single bond. More preferably, two or three among Y_{1b} to Y_{7b} represent each single bond, and the remains represent the other groups than single bond.

### (c) Substituted guanidine compound represented by the general formula:

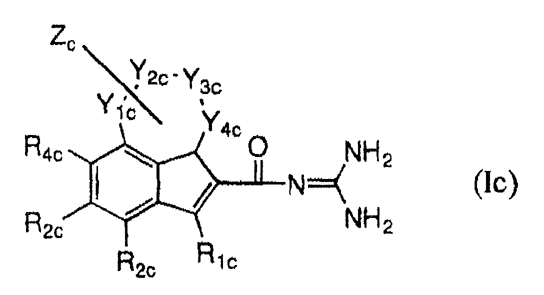

wherein R_{1c} represents a hydrogen atom, an alkyl group, a substituted alkyl group, a cycloalkyl group, a cycloalkenyl group, a saturated heterocyclic group, an aromatic group, an acyl group, or a group -OR_{5c} or -Q_{c}-R_{6c},
in which R_{5c} represents a hydrogen atom, an alkyl group, a substituted alkyl group, a cycloalkyl group, a cycloalkenyl group, a saturated heterocyclic group or an aromatic group;
Q_{c} represents optionally substituted lower alkylene group;
R_{6c} represents an optionally substituted vinyl group or an optionally
substituted ethynyl group;
R_{2c}, R_{3c} and R_{4c} represent independently a hydrogen atom, a nitro group , a carboxy group, an alkyl group, a substituted alkyl group, a cycloalkyl group, a cycloalkenyl group, a saturated heterocyclic group, an alkoxycarbonyl group, an aromatic group, an acyl group or a group -OR_{5c}, -N(R_{7c})R_{8c}, -CON(R_{7c})R_{8c}, -SO₂N(R_{7c})R_{8c}, -S(O)ₙR_{9c}, -Q_{c}-R_{6c} or the formula: 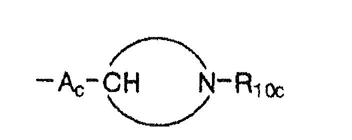 wherein R_{7c} and R_{8c} represent independently a hydrogen atom, an alkyl group, a substituted alkyl group, a cycloalkyl group, a cycloalkenyl group, a saturated heterocyclic group, an aromatic group, an acyl group or a group -Q_{c}-R_{6c}, or R_{7c} and R_{8c} are combined together to form with the nitrogen atom to which they are combined 5 to 7 membered saturated cyclic amino group optionally containing oxygen atom or sulfur atom in the ring, in which the cyclic amino group may be substituted with hydroxy group, alkyl group, substituted alkyl group or a group -OR_{5c};
R_{9c} represents an alkyl group, a substituted alkyl group or an aromatic group;
R_{10c} represents a hydrogen atom, an alkyl group, a substituted alkyl group, an acyl group or a group -Q_{c}-R_{6c};
the ring represents 3 to 8 membered saturated heterocyclic group consisting of a nitrogen atom and carbon atoms;
A_{c} represents oxygen atom, -S(O)ₙ- or -N(R_{11c})-, where R_{11c} represents a hydrogen atom, an alkyl group, a substituted alkyl group, a cycloalkyl group, a saturated heterocyclic group, an aromatic group, an acyl group or a group -Q_{c}-R_{6c};
Y_{1c}, Y_{2c}, Y_{3c} and Y_{4c} are (i) any one of them represents a methylene group, a carbonyl group, an oxygen atom, -S(O)ₙ-, -N(R_{11c})- or -C(=C(R_{12c})(R_{13c}))-,
wherein R_{12c} and R_{13c} represent independently a hydrogen atom, a halogen atom, a carboxy group, an alkyl group, a substituted alkyl group, an alkenyl group, an alkynyl group, a cycloalkyl group, a cycloalkenyl group, a saturated heterocyclic group, an alkoxycarbonyl group, an aromatic group, an acyl group, or a group -OR_{5c}, -N(R_{7c})R_{8c}, -CON(R_{7c})R_{8c}, -S(O)ₙR_{9c} or -Q_{c}-R_{6c}; and
n represents 0, 1 or 2,
any other two represent methylene groups, and the remaining one represents a single bond or a methylene group, or (ii) any adjacent two represent together a vinylene group (-CH=CH-) or -CON(R_{11c})-, any other one represents a methylene group, a carbonyl group, an oxygen atom, -S(O)n-, -N(R_{11c})- or -C(=C(R_{12c})(R_{13c}))-, and the remaining one represents a single bond or methylene group, provided that oxygen atom, nitrogen atom and sulfur atom do not combine directly to the vinylene group;
Z_{c} means a substituent which optionally substitute with one or more hydrogen atoms attaching to the carbon atoms constituting the ring, and each may be independently selected from the groups consisting of a halogen atom, a carboxy group, an alkyl group, a substituted alkyl group, an alkenyl group, an alkynyl group, a cycloalkyl group, a cycloalkenyl group, a saturated heterocyclic group, an alkoxycarbonyl group, an aromatic group or an acyl group and groups -OR_{5c}, -N(R_{7c})R_{8c}, -CON(R_{7c})R_{8c}, -S(O)ₙR_{9c} or -Q_{c}-R_{6c},
or pharmaceutically acceptable salt thereof.

With regard to this compound (Ic), the alkyl group may be linear or branched C₁-C₈ alkyl groups such as methyl, ethyl, propyl, 2-propyl, butyl, 2-butyl, 2-methylpropyl, 1,1-dimethylethyl, pentyl, hexyl, heptyl or octyl.

The cycloalkyl group may be a unsubstituted or a substituted with 1 to 4 hydroxy groups, alkyl groups, substituted alkyl groups or -OR_{5c} groups, particularly 3 to 8 membered cycloalkyl groups such as cyclopropyl, cyclobutyl, cyclopentyl, cyclohexyl, cycloheptyl, 2-methylcyclopentyl, 3-methylcyclopentyl, 2-methylcyclohexyl, 3-methylcyclohexyl, 4-methylcyclohexyl, 2-hydroxycyclopentyl, 3-hydroxycyclopentyl, 2-hydroxycyclohexyl, 3-hydroxycyclohexyl, 4-hydroxycyclohexyl, 2-(hydroxymethyl)cyclopentyl, 3-(hydroxymethyl)cyclopentyl, 2-(hydroxymethyl)cyclohexyl, 3-(hydroxymethyl)cyclohexyl, 4-(hydroxymethyl)cyclohexyl, 2-(aminomethyl)cyclopentyl, 3-(aminomethyl)cyclopentyl, 2-(aminomethyl)cyclohexyl, 3-(aminomethyl)cyclohexyl, 4-(aminomethyl)cyclohexyl, 2-(methoxymethyl)cyclopentyl, 3-(methoxymethyl)cyclopentyl, 2-(methoxymethyl)cyclohexyl, 3-(methoxymethyl)cyclohexyl or 4-(methoxymethyl)cyclohexyl.

The cycloalkenyl group may be a 3 to 8 membered cycloalkenyl group containing a double bond, which may be unsubstituted or substituted with 1 to 4 hydroxy groups, alkyl groups, substituted alkyl groups or -OR_{5c} groups, for example, 1-cyclopentenyl, 2-cyclopentenyl, 3-cyclopentenyl, 1-cyclohexenyl, 2-cyclohexenyl or cyclohexenyl.

The saturated heterocyclic group may be a 3 to 8 membered saturated heterocyclic group containing one of oxygen atom or sulfur atom, which may be unsubstituted or substituted with 1 to 4 hydroxy groups, alkyl groups, substituted alkyl groups or -OR_{5c} groups, particularly for example 2-tetrahydrofuryl, 3-tetrahydrofuryl, 2-tetrahydro-2H-pyranyl or 4-tetrahydro-4H-pyranyl.

The halogen atom may be, for example, fluorine, chlorine, or bromine atoms.

The alkoxycarbonyl group may be linear or branched C₂-C₆ alkoxycarbonyl groups such as methoxycarbonyl, ethoxycarbonyl, propoxycarbonyl or 2-propoxycarbonyl.

The aromatic group includes optionally substituted aryl groups and optionally substituted hetero-aryl groups. The aryl group may be C₆-C₁₀ aryl groups such as phenyl or naphthyl, and the hetero-aryl group may be a 5 or 6 membered hetero-aryl groups containing 1 to 4 nitrogen atoms or a 5 or 6 membered hetero-aryl group containing 0 to 2 nitrogen atoms and one of oxygen atom or a sulfur atom, such as 2-, 3-, 4-pyridyl, pyrrolyl, imidazolyl, triazolyl, tetrazolyl, 2-, 3-furyl, 2-, 3-thienyl, 1-, 3-, 4-oxazolyl, or 3-, 4- or 5-isoxazolyl.

The substituent in the substituted aryl group and the substituted hetero-aryl group includes a halogen atom, a nitro group, a carboxy group, an alkyl group, a substituted alkyl group, an alkoxycarbonyl group, and groups represented by the formula -OR_{5c}, -NR_{7c}R_{8c}, -CONR_{7c}, R_{8c}, -SO₂NR_{7c}R_{8c}, or -S(O)ₙR_{9c}.

Representative examples of the group -OR_{5c} in the case where R_{1c}, R_{2c}, R_{3c} and R_{4c} are the group of the formula -OR_{5c} in which R_{5c} is an aryl group are phenoxy group and substituted phenoxy group. The substituted phenoxy group may be a phenoxy groups substituted with, for example, a nitro group, -NR_{7c}R_{8c} group (where R_{7c} and R_{8c} may be for example a hydrogen atom or an alkyl group), or substituted alkyl group (where the substituent may be for example hydroxyl group or -NR_{7c}R_{8c} group), or the like.

Particularly the substituted phenoxy group may be for example o-, m- or p-nitrophenoxy, o-, m- or p-aminophenoxy, o-, m- or p-(dimethylamino)phenoxy, o-, m- or p-(aminomethyl)phenoxy, or o-, m- or p-(dimethylamino)phenoxy.

The alkoxy group may be linear or branched C₁-C₆ alkoxy groups such as methoxy, ethoxy, isopropoxy or tert-butoxy.

The 5 to 7 membered saturated cyclic amino group which is formed by combination of R_{7c} andR_{8c} together with the nitrogen atom to which they are combined and which optionally contains another hetero atom in the ring may be, for example, a 5 to 7 membered cyclic group containing 1 to 3 nitrogen atoms or a 5 to 7 membered cyclic group containing one of nitrogen atom and one of oxygen atom, and more particularly may be 1-pyrrolidinyl, 1-piperidino, 1-piperazinyl, 4-morpholino or 1-(4-methyl)piperazinyl.

The substituent in the substituted alkyl group includes a halogen atom, a hydroxyl group, a cyano group, a carboxy group, an alkoxy group, a cycloalkyl group, an alkoxycarbonyl group, an aromatic group, an acyl group and a group represented by the formula -CONR_{pc}R_{qc},
in which R_{pc} and R_{qc} represent independently each a hydrogen atom or an alkyl group, or R_{pc} and R_{qc} are combined together to form a 5 to 7 membered saturated cyclic amino group optionally containing another hetero atom,
or the formula -NR_{7c}R_{8c}, or the formula: 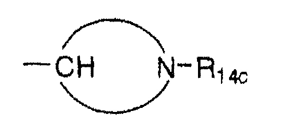 wherein R_{14c} represents a hydrogen atom, an alkyl group or a substituted alkyl group, and the ring represents 3 to 8 membered saturated heterocyclic ring containing a nitrogen atom.

Particularly the substituent in the substituted alkyl group for R_{1c}, R_{2c}, R_{3c}, R_{4c}, R_{5c}, R_{9c}, R_{10c}, R_{13c} and Z_{c} may be a halogen atom, a hydroxyl group, a carboxy group, a cycloalkyl group, an alkoxy group, an alkoxycarbonyl group, an acyl group, an aromatic group or a group represented by the formula -CONR_{pc}R_{qc} or -NR_{7c}R_{8c}, and the substituent in the substituted alkyl group for R_{7c}, R_{8c}, R_{11c}, R_{12c} and R_{14c} may be a hydroxyl group, a carboxy group, an cycloalkyl group, an alkoxy group, an alkoxycarbonyl group, an acyl group, an aryl group or a group represented by the formula -CONR_{pc}R_{qc} or -NR_{pc}R_{qc}. The alkyl moiety of the substituted alkyl group may be the same as those illustrated for the alkyl group in the above.

Such a substituted alkyl group may be, for example, a C₁-C₅ alkyl group substituted with C₃-C₆ cycloalkyl, a C₁-C₅ poly-haloalkyl group, a C₁-C₆ hydroxyalkyl group, a C₂-C₆ alkoxyalkyl group, a C₂-C₆ cyanoalkyl group, a C₂-C₆ carboxyalkyl group, a C₃-C₈ alkoxycarbonylalkyl group, a C₃-C₈ alkanoylalkyl group or C₈-C₁₆ aroylalkyl group, or optionally substituted phenyl- or naphthyl-C₁-C₅ alkyl group, or a carbamoyl-C₁-C₃ alkyl group in which the nitrogen atom may be optionally substituted with one or two C₁-C₃ alkyl, or amino-C₁-C₅ alkyl groups in which the nitrogen atom may be optionally substituted with one or two C₁-C₃ alkyl or with C₇-C₁₁ aralkyl, or 5 to 7 membered saturated cyclic amino-C₁-C₃ alkyl group, or the like.

Representative substituted alkyl group may be C₁-C₃ poly-haloalkyl groups such as trifluoromethyl, trifluoroethyl or trichloromethyl, or C₁-C₆ hydroxyalkyl groups such as hydroxymethyl, hydroxyethyl or 1-hydroxyethyl, or C₁-C₅ aminoalkyl groups such as aminomethyl, aminoethyl or 1-aminoethyl, or C₁-C₆ alkoxyalkyl groups such as methoxyethyl, ethoxyethyl or methoxypropyl, or C₂-C₆ carboxyalkyl groups such as carboxyethyl or carboxypropyl, or C₃-C₇ alkoxycarbonylalkyl groups such as methoxycarbonylmethyl, ethoxycarbonylmethyl or methoxycarbonylethyl, or phenyl- or naphthyl-C₁-C₅ alkyl groups such as benzyl, phenylethyl, phenylpropyl, phenylbutyl, or 1- or 2-naphthylmethyl, wherein the phenyl or naphthyl moiety may have substituent such as a halogen atom, a nitro group, an amino group, a hydroxyl group, a C₁-C₃ alkyl group or a C₁-C₃ alkoxy group, or a carbamoyl-C₁-C₃ alkyl group in which the nitrogen atom may be optionally substituted with one or two C₁-C₃ alkyl such as carbamoylmethyl, carbamoylethyl or dimethylcarbamoylmethyl, or an amino-C₁-C₅ alkyl group in which the nitrogen atom may be optionally substituted with one or two C₁-C₃ alkyl or C₇-C₁₁ aralkyl such as aminoethyl, aminopropyl, dimethylaminoethyl, dimethylaminopropyl, diethylaminoethyl or N-methyl-N-benzylaminoethyl, or a 5 to 7 membered saturated cyclic amino-C₁-C₃ alkyl groups such as 1-pyrrolidinylethyl, piperidinoethyl, or the like. Phenyl-C₁-C₅ alkyl group etc. can be mentioned for R_{7c} and R_{8c}.

The substituent in the lower alkylene group for Q_{c} and in the vinyl group and ethynyl group for R_{6c} may be, for example, a carboxy group, an alkyl group, a substituted alkyl group, a cycloalkyl group, a cycloalkenyl group, a saturated heterocyclic group, an alkoxycarbonyl group, an aromatic group or a group represented by the formula -CON(R_{7c})R_{8c}, or the like.

The lower alkylene group may be C₁-C₆ alkylene group such as methylene, ethylene, trimethylene, tetramethylene, pentamethylene or hexamethylene.

The acyl group may be C₁-C₆ alkanoyl groups such as formyl, acetyl or propanoyl, or C₃-C₆ cycloalkanecarbonyl groups such as cyclopropanecarbonyl, cyclobutanecarbonyl, cyclopentanecarbonyl or cyclohexanecarbonyl, or C₃-C₆ cycloalkenecarbonyl such as cyclopentenecarbonyl or cyclohexenecarbonyl, or C₇-C₁₁ aroyl groups such as benzoyl, toluoyl or naphthoyl, or saturated heterocyclic-carbonyl groups having a 5- or 6-membered saturated heterocyclic ring containing 1-2 hetero atoms selected from a nitrogen atom, an oxygen atom and a sulfur atom such as 2-piperidinecarbonyl or 3-morpholinecarbonyl, or aromatic heterocyclic acyl groups having a 5- or 6-membered aromatic heterocyclic ring containing 1-2 hetero atoms selected from a nitrogen atom, an oxygen atom and a sulfur atom, such as furoyl, thenoyl, nicotinoyl, isonicotinoyl, or the like.

The 5 to 7 membered saturated cyclic amino group which is formed by combination of R_{pc} and R_{qc} and optionally contains another hetero atom in the ring may be the same as the above cyclic amino groups formed by R_{7c} and R_{8c}.

The group represented by the formula -S(O)ₙR_{9c} may be C₁-C₈ alkanesulfonyl groups such as methanesulfonyl group, ethanesulfonyl group, propanesulfonyl group or isopropanesulfonyl group, and the corresponding alkanesulfinyl group and alkylthio group.

The group represented by the formula: 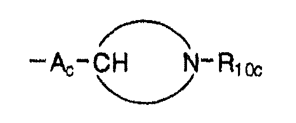 may be a group represented for example by the formula: 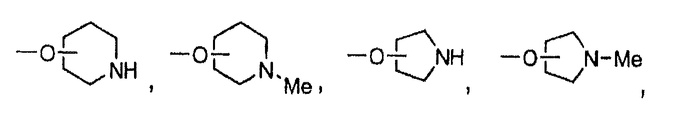 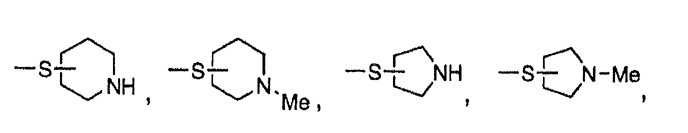 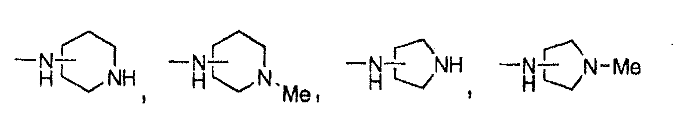 preferably may be (piperidin-3-yl)oxy, (piperidin-4-yl)oxy, (1-methylpiperidin-3-yl)oxy, (1-methylpiperidin-4-yl)oxy, (pyrrolidin-3-yl)oxy, (1-methylpyrrolidin-3-yl)oxy, (piperidin-3-yl)thio, (piperidin-4-yl)thio, (1-methylpiperidin-3-yl)thio, (1-methylpiperidin-4-yl)thio, (pyrrolidin-3-yl)thio, (1-methylpyrrolidin-2-yl)thio, (piperidin-3-yl)amino, piperidin-4-yl)amino, (1-methylpiperidin-3-yl)amino, (1-methylpiperidin-4-yl)amino, (pyrrolidin-3-yl)amino or (1-methylpyrrolidin-3-yl)amino.

The alkenyl group may be C₂-C₆ alkenyl groups such as vinyl, allyl, propenyl, 2-propenyl, butenyl, pentenyl or hexenyl.

The alkynyl group may be C₂-C₆ alkynyl groups such as ethynyl, propargyl, butynyl or pentynyl.

In addition to the above compounds, the following compounds (d), (e) and (f) and their pharmaceutically acceptable salts can be suitably used in the present invention.

### (d) Compounds represented by the formula (Id):

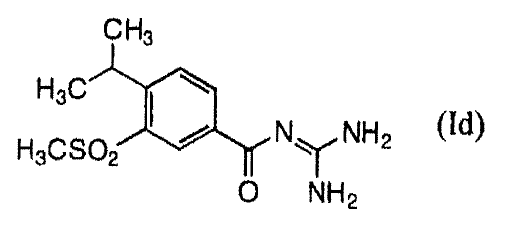

or salts thereof, particularly methanesulfonic acid salt.

### (e) Compounds represented by the formula (Ie):

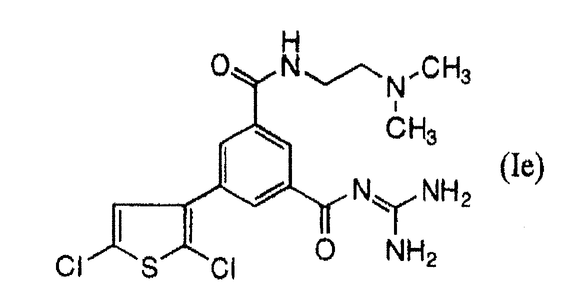

or salts thereof, particularly dihydrochloride.

### (f) Compounds represented by the formula (If):

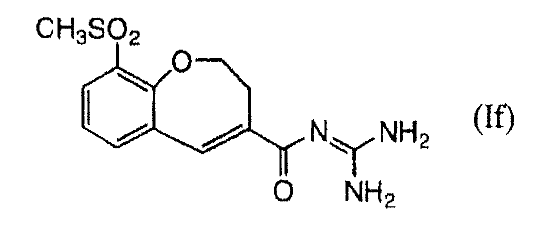

or salts thereof, particularly methanesulfonic acid salt.

The guanidine moiety of the above compounds (Ia) to (If) is in an equilibrium state represented by the following scheme: 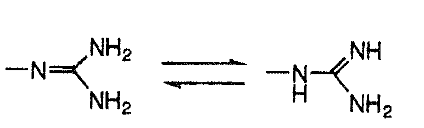 and the compounds used in the present invention include both of the tautomers.

The compounds represented by the general formulas (Ia), (Ib) and (Ic) include the ones having a center of optical asymmetry, and those compounds can be obtained as a racemic mixture or as one of the optical isomers when an optically active starting material is used. If necessary, the racemic mixture thus obtained can be physically or chemically divided into its optical antipodes by a known method. Preferably, a diastereoisomer is formed from the racemic mixture by a reaction using an agent for optical resolution. Diastereoisomers in different forms can be divided by a known method, for example by fractional crystallization.

The above compounds (Ia) to (If) can be converted to a pharmaceutically acceptable acid addition salt with an inorganic or organic acid, if necessary. Such an acid addition salt may be, for example, a salt with a mineral acid such as hydrochloric acid, hydrobromic acid, sulfuric acid or phosphoric acid.; a salt with an organic acid such as formic acid, acetic acid, fumaric acid, maleic acid, oxalic acid, citric acid, malic acid, tartaric acid, aspartic acid or glutamic acid.; a salt with a sulfonic acid such as methanesulfonic acid, benzenesulfonic acid, p-toluenesulfonic acid, hydroxybenzenesulfonic acid or dihydroxybenzenesulfonic acid.

Further, the compounds (Ia) to (If) and their acid addition salts may be anhydride, hydrate or solvate thereof.

In addition to the above compounds (Ia) to (If), the compounds having the activity for inhibiting Na⁺/H⁺ exchange transporter of the present invention may be, for example, the compounds described in Japanese Patent Kokai No. Hei 7-10839, Japanese Patent Kokai No. Hei 8-208602, Japanese Patent Kokai No. Hei 9-268172, Japanese Patent Kokai No. Hei 9-291076, Japanese Patent Kokai No. Hei 9-227496, Japanese Patent Kokai No. Hei 9-221465, Japanese Patent Kokai No. Hei 9-169719, Japanese Patent Kokai No. Hei 9-169718, Japanese Patent Kokai No. Hei 9-169721, Japanese Patent Kokai No. Hei 9-169723, Japanese Patent Kokai No. Hei 9-124584, Japanese Patent Kokai No. Hei 9-52823, Japanese Patent Kokai No. Hei 9-31045, Japanese Patent Kokai No. Hei 8-319266, Japanese Patent Kokai No. Hei 8-311012, Japanese Patent Kokai No. Hei 8-259515, Japanese Patent Kokai No. Hei 8-225514, Japanese Patent Kokai No. Hei 8-99950, Japanese Patent Kokai No. Hei 8-92215, Japanese Patent Kokai No. Hei 8-12643, Japanese Patent Kokai No. Hei 8-27093, Japanese Patent Kokai No. Hei 7-304729, Japanese Patent Kokai No. Hei 7-291927, Japanese Patent Kokai No. Hei 7-224022, Japanese Patent Kokai No. Hei 7-109251, Japanese Patent Kokai No. Hei 7-76566, Japanese Patent Kokai No. Hei 7-89938, Japanese Patent Kokai No. Hei 6-345715, Japanese Patent Kokai No. Hei 6-345643, Japanese Patent Kokai No. Hei 6-256291, Japanese Patent Kokai No. Hei 6-234730, Japanese Patent Kokai No. Hei 6-256290, Japanese Patent Kokai No. Hei 6-239828, Japanese Patent Kokai No. Hei 6-228082, Japanese Patent Kokai No. Hei 6-116230, Japanese Patent Kokai No. Hei 5-339228, Japanese Patent Kokai No. Hei 8-291131, Japanese Patent Kokai No. Hei 8-41028, Japanese Patent Kokai No. Hei 7-206823, Japanese Patent Kokai No. Hei 7-82234, Japanese Patent Kokai No. Hei 7-145149, Japanese Patent Kokai No. Hei 9-124583, Japanese Patent Kokai No. Hei 9-52876, Japanese Patent Kokai No. Hei 8-311011, Japanese Patent Kokai No. Hei 8-245560, Japanese Patent Kokai No. Hei 8-269001, Japanese Patent Kokai No. Hei 8-283232, Japanese Patent Kokai No. Hei 8-73427, Japanese Patent Kokai No. Hei 8-59598, Japanese Patent Kokai No. Hei 8-59602, Japanese Patent Kokai No. Hei 8-188568, Japanese Patent Kokai No. Hei 8-27113, Japanese Patent Kokai No. Hei 7-267926, Japanese Patent Kokai No. Hei 8-225513, Japanese Patent Kokai No. Hei 9-77753, Japanese Patent Kokai No. Hei 9-59245, Japanese Patent Kokai No. Hei 9-67332, Japanese Patent Kokai No. Hei 9-67340, Japanese Patent Kokai No. Hei 8-277269, Japanese Patent Kohyo No. Hei 6-509798, Japanese Patent Kohyo No. Hei 9-505035, Japanese Patent Kohyo No. Hei 9-504535, Japanese Patent Kohyo No. Hei 8-511243, Japanese Patent Kohyo No. Hei 9-500895, European Patent No.794172, European Patent No.794171, European Patent No.790245, European Patent No.765868, European Patent No.787728, German Patent No.19548708, German Patent No.19601303, WO 97/11055, WO 96/40728, WO 96/04241, WO 97/25310, WO 97/27183.

The above compounds (Ia) to (If) are described in the following patents, respectively. compounds (Ia): Japanese Patent Kokai No. Hei 8-208602, compounds (Ib): European Patent No.787728, compounds (Ic): Japanese Patent Kokai No. Hei 9-291076, compounds (Id): Japanese Patent Kokai No. Hei 6-228082, compounds (Ie): Japanese Patent Kohyo No. Hei 10-503770, compounds (If): WO 99/55690.

These substituted guanidine compounds and their pharmaceutically acceptable salts can be prepared by the methods described in each patent, respectively.

The therapeutic agent for frequent urination and urinary incontinence of the present invention contains as an active ingredient a substituted guanidine compound having inhibiting activity against Na⁺/H⁺ exchange transporter or its pharmaceutically acceptable salt.

The formulation of the treating agent is not limited and it may be, for example, injection type or non-injection type. In case of an injection, the above active ingredient may be dissolved in distilled water or a buffer or it may be lyophilized. If desired, the active ingredient may be formulated with, for example, solubilizing agent, emulsifying agent, or any other additive into a solution, suspension or emulsion. The solvent which can be used may be, for example, distilled water, physiological salt solution, alcohol such as ethanol, propanol, a sugar solution such as glucose solution or mannitol solution. or a mixture of these solvents.

The composition for treating frequent urination and/or urinary incontinence of the present invention may further contain another pharmaceutical additive such as surface active agent, emulsifying agent and stabilizing agent, if necessary. The formulation for injection contains an active ingredient in a concentration of, for example, about 0.1-10 % by weight.

In case of the formulation for non-injection, an active ingredient can be mixed with additive, for example, with excipient, stabilizing agent, diluent, binding agent, and then formulated by a conventional method into a proper administering form, for example, tablet, capsule, granulate, powder, syrup, suspension. The excipient may be, for example, gum arabic, magnesia, magnesium carbonate, calcium phosphate, lactose, glucose or starch, particularly corn starch.

The dose and administering frequency are preferably decided by taking account of the age, body weight, pathologies of the patient and the administering route, but usually 0.1-2000 mg, preferably 1-200 mg of the active ingredient is administered for an adult in one time to several times in one day.

The present invention provides a use of the substituted guanidine compounds or their pharmaceutically acceptable salts according to the present invention as a treating agent for frequent urination and/or urinary incontinence.

Further, the present invention provides a method for treating frequent urination and urinary incontinence by administering an effective amount of a guanidine compound or its pharmaceutically acceptable salt according to the present invention to a patient suffered from the above-mentioned diseases.

### Example

It should be understood that the following Examples and Test Examples are given only for explaining the present invention in more detail and that the present invention is not limited by these Examples.

In the following Test Examples and Examples, methanesulfonic acid salt of 4-isopropyl-3-methylsulfonylbenzoylguanidine (methanesulfonic acid salt of the compound obtained in Example 22 of Japanese Patent Kokai No. Hei 6-228082), i.e., methanesulfonic acid salt of a compound of the formula (Id) was used as a test compound in Test

### Example 1.

[5-(2,5-Dichlorothiofen-3-yl)-3-[(2-dimethylaminoethyl)carbamoyl] benzoyl]guanidine dihydrochloride (compound obtained in Example 8-(13) of Japanese Patent Kohyo No. Hei 10-503770), i.e., dihydrochloride of a compound of the formula (Ie) was used as a test compound in Test Example 2.

(2,3-Dihydro-9-methanesulfonyl-1-benzoxepine-4-carbonyl)-guanidine hydrochloride (compound obtained in Example 6 of WO 99/55690), i.e. methanesulfonic acid salt of a compound of the formula (If) was used as a test compound in Test Example 3.

### Test Example 1

Male SD rats (7 to 10 weeks old, Japan SLC) were anesthetized with urethane (1 to 1.2 g/kg, intraperitoneal administration) and their lower abdomen were opened along the midline. After performing a small dissection at the apex of the bladder, a cannula (Hibiki, size 5) attached with a balloon of about 1 ml volume was inserted into the bladder. A cannula for drug administration (PE50, Becton Dickinson) was inserted into the femoral vein. The cannula for measuring intravesical pressure was connected to a pressure transducer attached with a three-way cock and water was injected into the balloon from a free opening of the three-way cock to cause rhythmic bladder contraction. The intravesical pressure at that time was recorded by a thermal recorder (Recti-Horiz8K, NEC Sanei) via an amplifier (AP-601G, Nihon Kohden). The bladder contractile frequency and force were measured every 10 minutes before the administration of a test compound and for 0 to 10 and 10 to 20 minutes after the administration. Statistical analysis was performed using randomized block analysis of variance and Dunnett's multiple comparison with respect to the measurement values obtained before the administration.

A test compound 1 (4-isopropyl-3-methylsulfonylbenzoyl guanidine methanesulfonate) was intravenously administered to the rats at a dose of 3.2 mg/kg. In 0 to 10 minutes after the administration, the test compound 1 decreased the bladder contractile frequency to 25% of the value obtained before the administration without significantly affecting the bladder contractile force (p < 0.01).

### Test Example 2

In the same manner as Test Example 1, a test compound 2 ([5-(2,5-dichlorothiophene-3-yl)-3-[(2-dimethylaminoethyl)carbamoyl] benzoyl] guanidine dihydrochloride) was intravenously administered to the rats at a dose of 1 mg/kg. In 0 to 10 minutes after the administration, the test compound 2 decreased the bladder contractile frequency to 22% of the value obtained before the administration without significantly affecting the bladder contractile force (p < 0.05).

### Test Example 3

In the same manner as Test Example 1, a test compound 3 ((2,3-dihydro-9-methanesulfonyl-1-benzoxepin-4-carbonyl)guanidine dihydrochloride) was intravenously administered to the rats at a dose of 1 mg/kg. In 0 to 10 minutes after the administration, the test compound 3 decreased the bladder contractile frequency to 41% of the value obtained before the administration without significantly affecting the bladder contractile force (p < 0.05).

### Example 1

The following components were mixed by a conventional method to prepare powder formulation containing 1 mg of the above Test Compound 1 in one package.

| | |
|---|---|
| Test Compound 1 | 1 part by weight |
| Lactose | 39 parts by weight |

### Example 2

The following components were mixed by a conventional method to prepare powder formulation containing 1 mg of the above Test Compound 2 in one package.

| | |
|---|---|
| Test Compound 2 | 1 part by weight |
| Lactose | 39 parts by weight |

### Example 3

The following components were mixed by a conventional method to prepare powder formulation containing 1 mg of the above Test Compound 3 in one package.

| | |
|---|---|
| Test Compound 3 | 1 part by weight |
| Lactose | 39 parts by weight |

### Industrial applicability

The substituted guanidine compounds having an activity to inhibit Na⁺/H⁺ exchange transporter and pharmaceutically acceptable salts thereof possess an activity to suppress the frequency of bladder contractile without side effect such as dry mouth and accordingly they are useful as a medicament for treating frequent urination and/or urinary incontinence.

## Claims

1. A composition for treating frequent urination and/or urinary incontinence comprising a compound having an activity to inhibit Na⁺/H⁺ exchange transporter or its pharmaceutically acceptable salt as an active ingredient.

2. The composition for treating frequent urination and/or urinary incontinence according to Claim 1 wherein the compound having an activity to inhibit Na⁺/H⁺ exchange transporter or its salt is a compound represented by the following general formulas (Ia), (Ib), (Ic), (Id), (Ie) or (If) or its pharmaceutically acceptable salt:
(a) a substituted guanidine compound represented by the general formula (Ia) : 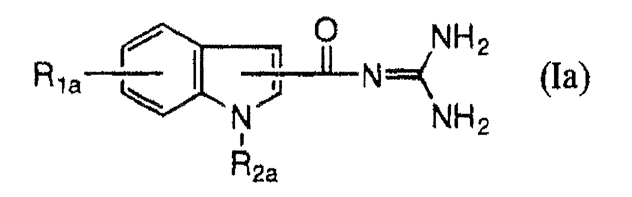 wherein R₁ₐ represents a hydrogen atom, a halogen atom, a nitro group, a carboxy group, an alkyl group, a substituted alkyl group, an alkenyl group, an alkynyl group, a cycloalkyl group, an acyl group, an alkoxycarbonyl group, an aromatic group, a group represented by the formula -OR₃ₐ, -NR₄ₐR₅ₐ, -SO₂NR₄ₐR₅ₐ or -S(O)ₙR₆ₐ,
in which R₃ₐ represents a hydrogen atom, an alkyl group, a substituted alkyl group, a cycloalkyl group, an aromatic group, or a group represented by the formula -CH₂R₇ₐ, where R₇ₐ represents a alkenyl group or a alkynyl group,
R₄ₐ and R₅ₐ represent independently a hydrogen atom, an alkyl group, a substituted alkyl group, a cycloalkyl group, an acyl group, an aromatic group or a group represented by the formula -CH₂R₈ₐ, where
R₈ₐ represents alkenyl group or an alkynyl group, or
R₄ₐ and R₅ₐ are combined together to form a 5 to 7 membered saturated cyclic amino group in which another hetero atom may be optionally contained,
R₆ₐ represents a alkyl group, a substituted alkyl group or an aryl group, and
n represents 0, 1 or 2,
or a group of the formula: 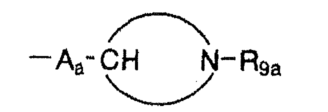 wherein the ring represents a 3 to 8 membered saturated heterocyclic ring containing a nitrogen atom and Aₐ represents an oxygen atom or a group of the formula -S(O)ₙ- or -N(R₁₀ₐ)-, in which R₁₀ₐ is hydrogen atom or alkyl group, and n is 0, 1 or 2;
R₉ₐ represents a hydrogen atom, an alkyl group or a substituted alkyl group, which may be further substituted with suitable substituent(s);
R₂ₐ represents a hydrogen atom, a hydroxy group, an alkyl group, a substituted alkyl group, a cycloalkyl group, an alkoxy group, an aromatic group, or a group represented by the formula -CH₂R₇ₐ, where R₇ₐ represents an alkenyl group or an alkynyl group;
the group R₁ₐ and guanidinocarbonyl group being optionally combined to either one of the 6-membered ring moiety and 5-membered ring moiety of the indole ring;
(b) a substituted guanidine compound represented by the general formula (Ib): 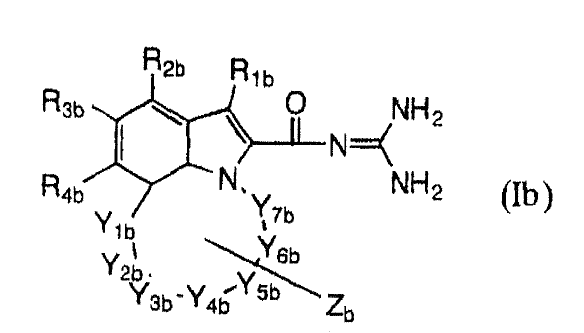 wherein R_{1b}, R_{2b}, R_{3b} and R_{4b} represent independently a hydrogen atom, a halogen atom, a nitro group, a carboxy group, an alkyl group, a substituted alkyl group, a cycloalkyl group, a cycloalkenyl group, a saturated heterocyclic group, an alkoxycarbonyl group, an aromatic group, an acyl group, or a group represented by the formula -OR_{5b}, -N(R_{6b})R_{7b}, -CON(R_{6b})R_{7b}, -SO₂N(R_{6b})R_{7b}, -S(O)ₙR_{8b}, -Q_{b}-R_{9b} or the formula: 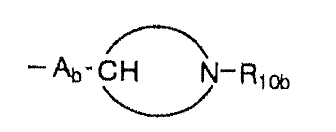 wherein R_{5b} represents a hydrogen atom, an alkyl group, a substituted alkyl group, a cycloalkyl group, a cycloalkenyl group, a saturated heterocyclic group or an aromatic group;
R_{6b} and R_{7b} represent independently a hydrogen atom, an alkyl group, a substituted alkyl group, a cycloalkyl group, a cycloalkenyl group, a saturated heterocyclic group, an aromatic group, an acyl group or a group -Q_{b}-R_{9b}, or
R_{6b} and R_{7b} are combined together to form with the nitrogen atom to which they are combined a 5 to 7 membered saturated cyclic amino group which may contain another hetero atom in the ring, in which the said cyclic amino group may be substituted with one or more hydroxy groups, alkyl groups, substituted alkyl groups, or a group -OR_{5b};
R_{8b} represents alkyl group, substituted alkyl group or aromatic group;
Q_{b} represents optionally substituted lower alkylene group;
R_{9b} represents optionally substituted vinyl group or optionally substituted ethynyl group;
A_{b} represents oxygen atom, -S(O)ₙ- or -N(R_{11b})-, wherein R_{11b} represents hydrogen atom, alkyl group, substituted alkyl group, cycloalkyl group, saturated heterocyclic group, aromatic group, acyl group or -Q_{b}-R_{9b};
R_{10b} represents a hydrogen atom, an alkyl group, a substituted alkyl group, an acyl group or -Q_{b}-R_{9b};
the ring represents 3 to 8 membered saturated heterocyclic group consisting of a nitrogen atom and carbon atoms;
Y_{1b}, Y_{2b}, Y_{3b}, Y_{4b}, Y_{5b}, Y_{6b} and Y_{7b} are the same or different and represent a single bond, -CH₂-, -O-, -CO-, -C(=C(R_{12b})R_{13b})-, -S(O)ₙ-, or -N(R_{11b})-,
wherein R_{12b} and R_{13b} represent independently a hydrogen atom, an alkyl group, a substituted alkyl group, an alkenyl group, an alkynyl group, a cycloalkyl group, a cycloalkenyl group, a saturated heterocyclic group, halogen atom, carboxy group, alkoxycarbonyl group, aromatic group, acyl or a group -OR_{5b}, N(R_{6b})R_{7b}, -CON(R_{6b})R_{7b}, -S(O)ₙR_{8b} Or -Q_{b}-R_{9b};
n is an integer of 0, 1 or 2, or
adjacent two groups represent together -CH=CH-, provided that among Y_{1b} to Y_{7b}, at least two of them represent group other than single bond;
Z_{b}, which may be absent or may be one or more and substitutes for hydrogen atom attaching to the carbon atom constituting the ring Y_{1b} to Y_{7b}, are the same or different, each represents an alkyl group, a substituted alkyl group, an alkenyl group, an alkynyl group, a cycloalkyl group, a cycloalkenyl group, a saturated heterocyclic group, a halogen atom, an carboxy group, an alkoxycarbonyl group, an aromatic group, an acyl group or a group -OR_{5b}, -N(R_{6b})R_{7b}, -S(O)ₙR_{8b}, -C(O)N(R_{6b})R_{7b} or -Q_{b}-R_{9b}, provided that Z_{b} is not -N(R_{6b})R_{7b} or -S(O)ₙR_{8b} when Z_{b} substitutes for the hydrogen atom of -CH=CH-;
(c) a substituted guanidine compound represented by the general formula: 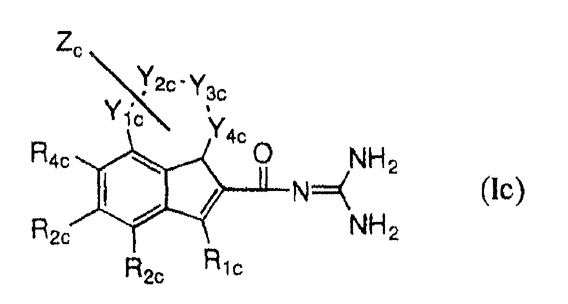 wherein R_{1c} represents a hydrogen atom, an alkyl group, a substituted alkyl group, a cycloalkyl group, a cycloalkenyl group, a saturated heterocyclic group, an aromatic group, an acyl group, or a group -OR_{5c} or -Q_{c}-R_{6c},
wherein R_{5c} represents a hydrogen atom, an alkyl group, a substituted alkyl group, a cycloalkyl group, a cycloalkenyl group, a saturated heterocyclic group or an aromatic group;
Q_{c} represents optionally substituted lower alkylene group;
R_{6c} represents an optionally substituted vinyl group or an optionally substituted ethynyl group;
R_{2c}, R_{3c} and R_{4c} represent independently a hydrogen atom, a nitro group , a carboxy group, an alkyl group, a substituted alkyl group, a cycloalkyl group, a cycloalkenyl group, a saturated heterocyclic group, an alkoxycarbonyl group, an aromatic group, an acyl group or a group -OR_{5c}, -N(R_{7c})R_{8c}, -CON(R_{7c})R_{8c}, -SO₂N(R_{7c})R_{8c}, -S(O)ₙR_{9c}, -Q_{c}-R_{6c} or the formula: 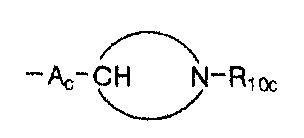 wherein R_{7c} and R_{8c} represent independently a hydrogen atom, an alkyl group, a substituted alkyl group, a cycloalkyl group, a cycloalkenyl group, a saturated heterocyclic group, an aromatic group, an acyl group or a group -Q_{c}-R_{6c}, or R_{7c} and R_{8c} are combined together to form with the nitrogen atom to which they are combined 5 to 7 membered saturated cyclic amino group optionally containing oxygen atom or sulfur atom in the ring, in which the cyclic amino group may be substituted with hydroxyl group, alkyl group, substituted alkyl group or a group -OR_{5c};
R_{9c} represents an alkyl group, a substituted alkyl group or an aromatic group;
R_{10c} represents a hydrogen atom, an alkyl group, a substituted alkyl group, an acyl group or a group -Q_{c}-R_{6c};
the ring represents 3 to 8 membered saturated heterocyclic group consisting of one of nitrogen atom and carbon atoms;
A_{c} represents an oxygen atom, -S(O)ₙ- or -N(R_{11c})-, where R_{11c} represents a hydrogen atom, an alkyl group, a substituted alkyl group, a cycloalkyl group, a saturated heterocyclic group, an aromatic group, an acyl group or a group -Q_{c}-R_{6c};
Y_{1c}, Y_{2c}, Y_{3c} and Y_{4c} are (i) any one of them represents a methylene group, a carbonyl group, an oxygen atom, -S(O)ₙ-, -N(R_{11c})- or -C(=C(R_{12c})(R_{13c}))-,
wherein R_{12c} and R_{13c} represent independently a hydrogen atom, a halogen atom, a carboxy group, an alkyl group, a substituted alkyl group, an alkenyl group, an alkynyl group, a cycloalkyl group, a cycloalkenyl group, a saturated heterocyclic group, an alkoxycarbonyl group, an aromatic group, an acyl group, or a group -OR_{5c}, -N(R_{7c})R_{8c}, -CON(R_{7c})R_{8c}, -S(O)ₙR_{9c} or -Q_{c}-R_{6c}; and
n represents 0, 1 or 2,
any other two represent methylene groups, and the remaining one represents a single bond or a methylene group, or (ii) any adjacent two represent together a vinylene group (-CH=CH-) or -CON(R_{11c})-, any other one represents a methylene group, a carbonyl group, an oxygen atom, -S(O)ₙ-, -N(R_{11c})- or -C(=C(R_{12c})(R_{13c}))-, and the remaining one represents a single bond or methylene group, provided that oxygen atom, nitrogen atom and sulfur atom do not combine directly to the vinylene group;
Z_{c} means a substituent which optionally substitute with one or more hydrogen atoms attaching to the carbon atoms constituting the ring, and each may be independently selected from the group consisting of a halogen atom, a carboxy group, an alkyl group, a substituted alkyl group, an alkenyl group, an alkynyl group, a cycloalkyl group, a cycloalkenyl group, a saturated heterocyclic group, an alkoxycarbonyl group, an aromatic group, an acyl group and group -OR_{5c}, -N(R_{7c})R_{8c}, -CON(R_{7c})R_{8c}, -S(O)ₙR_{9c} or -Q_{c}-R_{6c};
(d) a compound represented by the formula (Id): 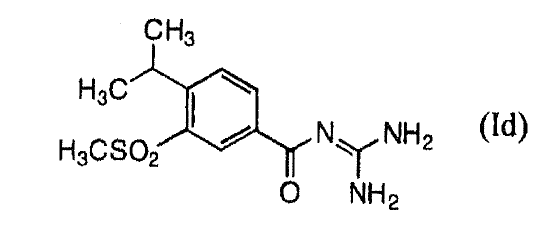
(e) a compound represented by the formula (Ie): 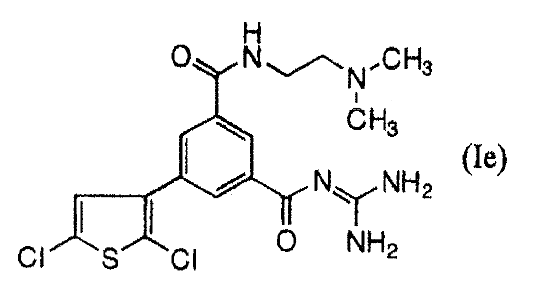
(f) a compound represented by the formula (If): 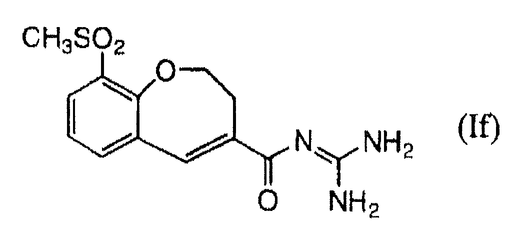 or a pharmaceutically acceptable salt thereof.

3. The composition for treating frequent urination and/or urinary incontinence according to Claim 2 wherein the compound having an activity to inhibit Na⁺/H⁺ exchange transporter or its pharmaceutically acceptable salt is a compound represented by the formula (Ia) or its pharmaceutically acceptable salt.

4. The composition for treating frequent urination and/or urinary incontinence according to Claim 2 wherein the compound having an activity to inhibit Na⁺/H⁺ exchange transporter or its pharmaceutically acceptable salt is a compound represented by the formula (Ib) or its pharmaceutically acceptable salt.

5. The composition for treating frequent urination and/or urinary incontinence according to Claim 2 wherein the compound having an activity to inhibit Na⁺/H⁺ exchange transporter or its pharmaceutically acceptable salt is a compound represented by the formula (Ic) or its pharmaceutically acceptable salt.

6. The composition for treating frequent urination and/or urinary incontinence according to Claim 2 wherein the compound having an activity to inhibit Na⁺/H⁺ exchange transporter or its pharmaceutically acceptable salt is a compound represented by the formula (Id) or its pharmaceutically acceptable salt.

7. The composition for treating frequent urination and/or urinary incontinence according to Claim 2 wherein the compound having an activity to inhibit Na⁺/H⁺ exchange transporter or its pharmaceutically acceptable salt is a compound represented by the formula (Ie) or its pharmaceutically acceptable salt.

8. The composition for treating frequent urination and/or urinary incontinence according to Claim 2 wherein the compound having an activity to inhibit Na⁺/H⁺ exchange transporter or its pharmaceutically acceptable salt is a compound represented by the formula (If) or its pharmaceutically acceptable salt.

## Amended claims

### Amended claims under Art. 19.1 PCT

1. A composition for treating frequent urination and/or urinary incontinence comprising a substituted guanidine compound having an activity to inhibit Na⁺/ H⁺ exchange transporter or its pharmaceutically acceptable salt as an active ingredient.

2. The composition for treating frequent urination and/or urinary incontinence according to Claim 1 wherein the compound having an activity to inhibit Na⁺/ H⁺ exchange transporter or its salt is a compound represented by the following general formulas (Ia), (Ib), (Ic), (Id), (Ie) or (If) or its pharmaceutically acceptable salt:
(a) a substituted guanidine compound represented by the general formula (Ia) 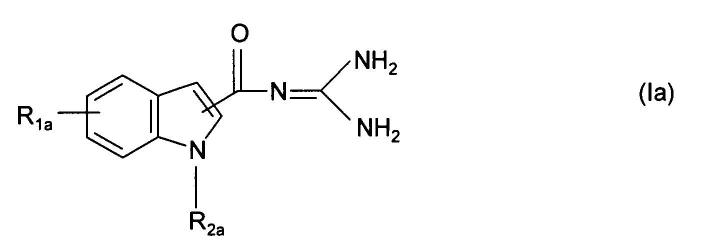 wherein R₁ₐ represents a hydrogen atom, a halogen atom, a nitro group, a carboxy group, an alkyl group, a substituted alkyl group, an alkenyl group, an alkynyl group, a cycloalkyl group, an acyl group, an alkoxycarbonyl group, an aromatic group, a group represented by the formula -OR₃ₐ, -NR₄ₐR₅ₐ, -SO₂NR₄ₐR₅ₐ or -S(O)ₙR₆ₐ,
in which R₃ₐ represents a hydrogen atom, an alkyl group, a substituted alkyl group, a cycloalkyl group, an aromatic group, or a group represented by the formula -CH₂R₇ₐ, where R₇ₐ represents a alkenyl group or a alkynyl group,
